# EUROPEAN PATENT APPLICATION

(11) **EP 4 082 494 A1**
(43) Date of publication of application: **02.11.2022**
(21) Application number: 20907727.0
(22) Date of filing: 20.08.2020
(51) Int. Cl.: A61F 7/03

(54) **HEATING IMPLEMENT**

(30) Priority: 25.12.2019 WO PCT/JP2019/050934
(71) Applicant: Kao Corporation, Chuo-ku Tokyo 103-8210 (JP)
(72) Inventor: TAKAKUWA, Hotaka, Tokyo 131-0044 (JP)
(74) Representative: Hoffmann Eitle
(86) International application number: PCT/JP2020/031436
(87) International publication number: WO 2021/131151

(57) **Abstract**

The present invention relates to a warming device (1) including a heat generating element (3) and an enclosing material (2) that houses the heat generating element (3). A portion of the enclosing material (2) that comes into contact with a target to be warmed when the warming device (1) is in use is made of an air permeable fibrous sheet that contains crimped fibers and non-crimped fibers. The ratio of the non-crimped fibers relative to the total of the crimped fibers and the non-crimped fibers in the fibrous sheet is 50 mass% or more. The fibrous sheet included in the heat generating element (1) is configured to increase the thickness thereof by 0.2 mm or more and 0.7 mm or less during a period from the start to the end of heat generation. Furthermore, the portion of the enclosing material (2) that comes into contact with the target to be warmed when the warming device (1) is in use may be made of an air permeable fibrous sheet that contains two or more of materials that have different melting points.

## Description

### Technical Field

The present invention relates to a warming device.

### Background Art

The applicant of the present application previously proposed an eye mask that includes a laterally oblong mask main body for covering eyes when the eye mask is worn and a pair of ear loop portions that are provided in the vicinity of laterally opposing outer edge portions of the mask main body and are placed around ears when the eye mask is worn (see Patent Literature 1). The mask main body disclosed in Patent Literature 1 includes a steam/warm heat generation material, and the steam and warm heat generated from the steam/warm heat generation material can be uniformly applied to the wearer wearing the eye mask.

### Citation List

### Patent Literature

Patent Literature 1: JP 2008-295779A

### Summary of Invention

The present invention relates to a warming device including a heat generating element and an enclosing material that houses the heat generating element.

According to one embodiment, a portion of the enclosing material that comes into contact with a target to be warmed when the warming device is in use is made of an air permeable fibrous sheet that contains crimped fibers and non-crimped fibers.

According to one embodiment, the ratio of the non-crimped fibers relative to the total of the crimped fibers and the non-crimped fibers is 50 mass% or more.

According to one embodiment, the heat generating element contains an oxidizable metal powder, a carbon material powder and water.

According to one embodiment, the heat generating element is configured to generate steam along with heat generation.

The present invention also relates to a warming device including a heat generating element and an enclosing material that houses the heat generating element.

According to one embodiment, a portion of the enclosing material that comes into contact with a target to be warmed when the warming device is in use is made of a fibrous sheet.

According to one embodiment, the fibrous sheet is configured to increase the thickness thereof by 0.2 mm or more and 0.7 mm or less during a period from a start to an end of heat generation by the warming device.

According to one embodiment, the heat generating element contains an oxidizable metal powder, a carbon material powder and water.

According to one embodiment, the heat generating element is configured to generate steam along with heat generation.

The present invention also relates to a warming device including a heat generating element and an enclosing material that houses the heat generating element.

According to one embodiment, a portion of the enclosing material that comes into contact with a target to be warmed when the warming device is in use is made of a fibrous sheet.

According to one embodiment, the fibrous sheet is configured to increase the thickness thereof by 0.10 mm or more and 0.60 mm or less during a period of 5 minutes after the start of heat generation by the warming device.

According to one embodiment, the heat generating element contains an oxidizable metal powder, a carbon material powder and water.

According to one embodiment, the heat generating element is configured to generate steam along with heat generation.

The present invention also relates to a warming device including a heat generating element and an enclosing material that houses the heat generating element.

According to one embodiment, a portion of the enclosing material that comes into contact with a target to be warmed when the warming device is in use is made of a fibrous sheet.

According to one embodiment, the fibrous sheet is configured to increase the thickness thereof by 0.15 mm or more and 0.70 mm or less during a period of 1 hour after a start of heat generation by the warming device.

According to one embodiment, the heat generating element contains an oxidizable metal powder, a carbon material powder and water.

According to one embodiment, the heat generating element is configured to generate steam along with heat generation.

### Brief Description of Drawings

[Fig. 1] Fig. 1 is a plan view of a warming device according to an embodiment of the present invention.
[Fig. 2] Fig. 2 is an exploded perspective view of the warming device shown in Fig. 1.
[Fig. 3] Fig. 3 is a cross-sectional view of the warming device shown in Fig. 1 taken along the lateral direction that is a longitudinal direction of the warming device.
[Fig. 4] Fig. 4 is an enlarged cross-sectional view of the warming device shown in Fig. 3.
[Fig. 5] Fig. 5 is a schematic diagram of an apparatus for measuring the amount of steam generated from the warming device.
[Fig. 6] Fig. 6 is a plan view of a warming device according to another embodiment of the present invention.

### Description of Embodiments

With an increase in diversification of consumer needs and required performances in recent years, demand for a warming device with more excellent levels of flexibility and fit is increasing. In particular, high levels of properties that keep flexibility and a fit throughout the period from immediately after the start of use of the warming device to the end of use of the warming device are required. The warming device disclosed in Patent Literature 1 can provide an improved fit to the body of the wearer, but there is still room for further improvement in terms of achieving higher levels of both flexibility and fit to the body of the wearer.

Accordingly, the present invention relates to a warming device with improved flexibility and fit to the body of the wearer.

Hereinafter, the present invention will be described based on a preferred embodiment thereof with reference to the drawings. The warming device according to the present invention is used to apply warm heat to a target to be warmed by being brought into contact with the target to be warmed when the warming device is in use.

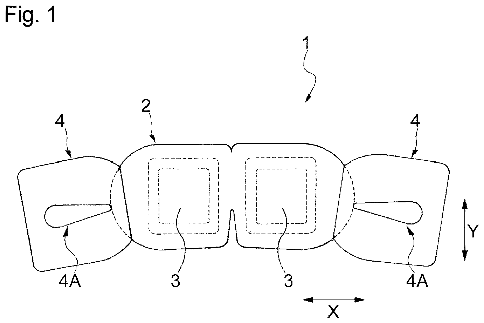

Fig. 1 shows a warming device according to an embodiment of the present invention. The warming device 1 shown in Fig. 1 is a so-called eye mask-type warming device, and is used by being brought into contact with human eyes that is a target to be warmed so as to cover the eyes when the warming device 1 is in use and apply warm heat to the eyes and the vicinity thereof. The warming device 1 is configured to generate steam heated to a predetermined temperature. With this configuration, warm heat can be applied to the target to be warmed.

As shown in Fig. 1, the warming device 1 includes: an enclosing material 2 that has a shape elongated in a lateral direction X and covers both eyes of the user when the warming device 1 is in use; a heat generating element 3 that is housed in the enclosing material 2; and a pair of ear loop portions 4. The ear loop portions 4 are provided in opposing outer end regions of the enclosing material 2 in the lateral direction X, and can be opened outward in the lateral direction X. With this configuration, by placing the ear loop portions 4 around the ears of the user, the eyes of the user can be kept covered with the enclosing material 2. From the viewpoint of improving the ease of wearing, an elastic sheet is preferably used as a sheet material that constitutes the ear loop portions 4. In the description given below, a direction that corresponds to the longitudinal direction of the warming device 1 will also be referred to as "lateral direction X", and a direction perpendicular to the lateral direction X will also be referred to as "length direction Y".

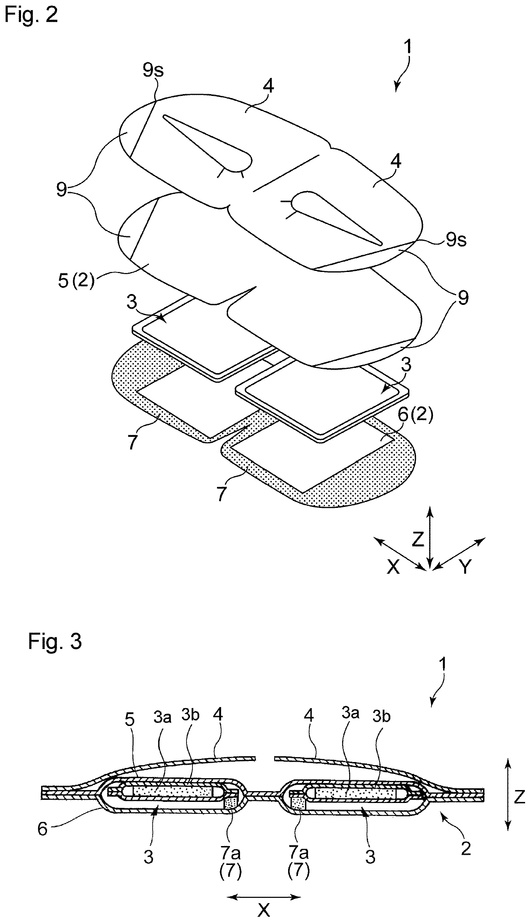

Fig. 2 shows an exploded perspective view of the warming device 1. Fig. 3 shows a cross-sectional view of the warming device 1 taken along the lateral direction X (longitudinal direction). The enclosing material 2 included in the warming device 1 shown in these diagrams is a flat enclosing material that includes a topsheet 5 that is located on a side that is close to the skin of the user and a backsheet 6 that is located on a side that is far from the skin of the user. The topsheet 5 constitutes a surface that includes a portion that comes into contact with a target to be warmed such as human eyes when the warming device 1 is in use. The backsheet 6 constitutes a surface that is far from the skin of the user, and forms an outer surface of the warming device 1. That is, in Figs. 2 and 3, the upper side indicates the side that is close to the skin of the user, and the lower side indicates the side that is far from the skin of the user.

The topsheet 5 and the backsheet 6 shown in Figs. 2 and 3 are superimposed and bonded to each other with an adhesive 7 such as a hot melt adhesive. Accordingly, two heat generating elements 3 spaced apart from each other in the lateral direction X are housed between the two sheets 5 and 6. It is preferable that, out of the topsheet 5 and the backsheet 6, at least the topsheet 5 is made of an air permeable fibrous sheet. A detailed description of the sheets 5 and 6 will be given later. The fibrous sheet is a collection of a plurality of constituent fibers that have been formed into the shape of a sheet by at least one of entanglement, fusion bonding, and bonding of the plurality of constituent fibers.

The heat generating elements 3 held between the topsheet 5 and the backsheet 6 are configured to react with oxygen in the air to generate heat and, along with the heat generation, generate steam heated to a predetermined temperature. That is, the heat generating elements 3 have a function of generating steam along with heat generation. More specifically, the heat generating elements 3 each include a heat generation portion 3a that contains an oxidizable metal that generates heat along with an oxidation reaction with oxygen in the air, a reaction accelerator such as a carbon material including activated carbon that serves as a catalyst for the oxidation reaction and water, and preferably further contains an electrolyte such as a metal salt. The oxidizable metal and the carbon material are both preferably in the form of a powder.

The heat generation portion 3a may be, for example, a heat generation sheet that is made of a fibrous sheet that contains an oxidizable metal, a reaction accelerator, fibrous material, an electrolyte and water, or may be made of a heat generation composition in the form of a paste or a powder that contains an oxidizable metal, a reaction accelerator, a liquid retainer such as a water absorbent polymer, an electrolyte and water. The heat generation sheet and the heat generation composition may be used alone, or at least one of the heat generation sheet and the heat generation composition may be housed in a pouch-like member formed by bonding a plurality of sheet materials together. As the materials that constitute the heat generation sheet and the heat generation composition, for example, the materials disclosed in JP 2003-102761A and JP 2006-340928A may be used.

The cross-sectional view of Fig. 3 shows a state in which flat heat generating elements 3 are fixed, each heat generating element 3 including a heat generation portion 3a that is housed in a pouch-like member 3b. In each of the heat generating elements 3 shown in Fig. 3, a portion of the outer surface of the pouch-like member 3b is fixed to the inner surface of the backsheet 6 of the warming device 1 with an adhesive fixing portion 7a that is made using an adhesive 7, and the remaining surface is not fixed to the backsheet 6. The adhesive fixing portions 7a are provided in a center region of the warming device 1 in the lateral direction X, and extend in the length direction Y of the warming device 1. With this configuration, when the warming device 1 is in use, the heat generating elements 3 are appropriately placed on a target to be warmed such as user's eyes and the vicinity of the user's eyes, and thus warm heat can be efficiently applied to the target to be warmed.

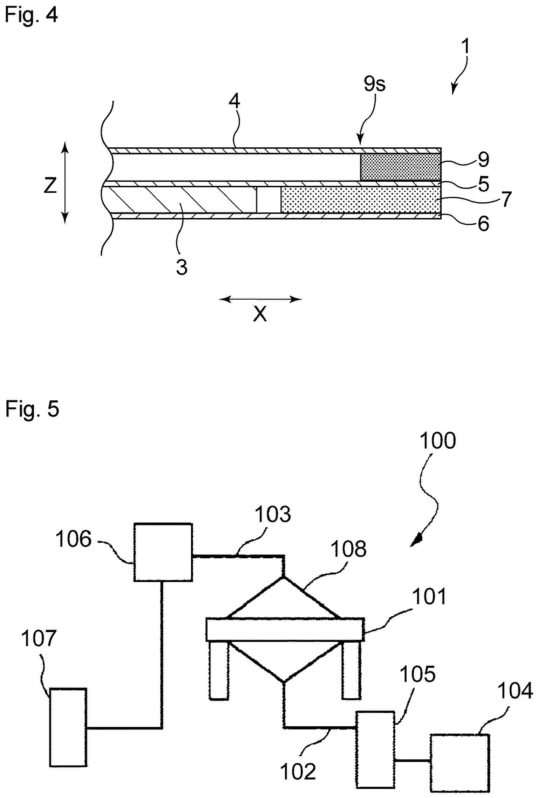

Returning to Fig. 2, the ear loop portions 4 shown in Fig. 2 are each made of a sheet material, and an insertion portion 4A extending in the lateral direction X is formed in the sheet material. The insertion portions 4A are holes through which the ears are passed when the ear loop portions 4 are placed around the ears. Instead of this configuration, the insertion portions 4A may be through slits or the like through which the ears can be passed. As shown in Figs. 2 and 4, the ear loop portions 4 are bonded to the outer surface of the topsheet 5 of the enclosing material 2 in opposing outer end regions of the ear loop portions 4 in the lateral direction X, as a result of which, bonding regions 9 are formed where the enclosing material 2 and the ear loop portions 4 are bonded. The bonding regions 9 also function as bending portions that are bent along bonding end portions 9s when the ear loop portions 4 are opened outward.

Fig. 4 is a cross-sectional view showing a configuration of a bonding region 9. Each bonding region 9 shown in Figs. 2 and 4 where the enclosing material 2 and an ear loop portion 4 are bonded has a semi elliptical shape, and a portion extending from the bonding end portion 9s that is the inner end of the bonding region 9 in the lateral direction X to the outer end of the enclosing material 2 in the lateral direction X is continuously bonded. As shown in Fig. 4, the bonding region 9 is formed as a result of the topsheet 5 and the ear loop portion 4 being bonded. The bonding region 9 also functions as a bending portion that is bent along the bonding end portion 9s when the ear loop portion 4 is opened outward. Each bonding region 9 shown in Figs. 2 and 4 is formed by being continuously bonded, but may be formed by being intermittently bonded.

The expression "to generate steam" used in the present disclosure means that the total amount of steam generated for 10 minutes measured using a method described below is 10 mg/10 min or more. This amount of steam generation can be easily achieved by using, for example, a paste that contains an oxidizable metal, a carbon material and water when forming the heat generation portion 3a.

The amount of steam generation can be measured using, for example, an apparatus 100 that has a configuration shown in Fig. 5. The apparatus 100 includes an aluminum measurement chamber 101 (volume: 4.2 L), a flow inlet path 102 that is in communication with a lower portion of the measurement chamber 101, and a flow outlet path 103 that is in communication with an upper portion of the measurement chamber 101. The flow inlet path 102 is configured to cause dehumidified air (humidity: less than 2%RH, and flow rate: 2.1 L/min) supplied from an air supply unit (not shown) to flow into the measurement chamber 101. In addition thereto, the apparatus 100 includes an inlet thermo hygrometer 104 and an inlet flow meter 105 that are provided to the flow inlet path 102, an outlet thermo hygrometer 106 and an outlet flow meter 107 that are provided to the flow outlet path 103, and a thermometer (thermistor) 108 that is provided in the measurement chamber 101. As the thermometer 108, it is preferable to use a thermometer with a temperature resolution of about 0.01°C.

A method for measuring the total amount of steam generation using the apparatus 100 is as follows.

First, as a measurement target, a warming device hermetically housed in an oxygen blocking pouch is prepared. Then, the oxygen blocking pouch is opened, and one heat generating element is taken out from the oxygen blocking pouch. In the case where the heat generating element is housed in a pouch-like member, the heat generating element is taken out together with the pouch-like member.

The heat generating element that has been taken out from the oxygen blocking pouch is placed in the measurement chamber 101 in such a manner that one side of the pouch-like member of the heat generating element faces outside, and then the thermometer 108 is placed thereon. In the case where one side and the other side of the pouch-like member are made of sheet materials that have different levels of air permeability, the heat generating element is placed in the measurement chamber 101 in such a manner that one side of the pouch-like member that is made of a sheet material that has a higher level of air permeability faces outside, and the thermometer 108 is placed on that side.

In this state, dehumidified air is caused to flow from the lower portion of the measurement chamber 101 via the flow inlet path 102, and the difference in absolute humidity between before and after the air flows through the measurement chamber 101 based on the temperature and the humidity measured by each of the inlet thermo hygrometer 104 and the outlet thermo hygrometer 106. Furthermore, the amount of steam discharged from the heat generating device is calculated based on the air flow rate measured by each of the inlet flow meter 105 and the outlet flow meter 107. The total amount of steam refers to the total amount (mg/10 min) of steam measured for 10 minutes from the start of measurement that is the time at which the warming device is taken out from the oxygen blocking pouch, and the heat generating element is exposed to the air.

In the warming device 1 configured as described above, the topsheet 5, which is a portion that comes into contact with a target to be warmed when the warming device 1 is in use, preferably has a predetermined fiber composition ratio. In the description given below, an example will be described in which the topsheet 5 is a fibrous sheet that is a preferable embodiment of the topsheet 5.

More specifically, the topsheet 5 is preferably a fibrous sheet that contains, as constituent fibers that constitute the topsheet 5, fibers that are crimped (hereinafter also referred to as "crimped fibers") and fibers that are not crimped (hereinafter also referred to as "non-crimped fibers"). Also, it is preferable that the fibrous sheet has air permeability, and it is more preferable that the fibrous sheet is a non-woven fabric. As a result of the topsheet 5 having the above-described configuration, the number of contacting parts between fibers can be reduced, and the volume of the sheet can be increased. Accordingly, a topsheet 5 with an excellent level of fit can be easily obtained.

In the case of an ordinary warming device that uses heat generated by an oxidation reaction of iron, the warming device before use is hermetically housed into a pouch that blocks outside air from entering the pouch in the absence of oxygen so as to prevent the warming device from coming into contact with oxygen and prevent an unintentional progress of the oxidation reaction of iron. In this case, even if the topsheet is configured at the time of production to exhibit flexibility and a fit, when or after the warming device including the topsheet is hermetically housed into the pouch, or when the warming device hermetically housed in the pouch is distributed, the topsheet is likely to be compressed in the thickness direction, as a result of which, the flexibility and the fit caused by the topsheet are unlikely to be exhibited as designed, and thus there is room for improvement. In this regard, the inventors of the present application conducted in-depth studies to improve the flexibility and the fit, and obtained the following findings. In the present embodiment, by configuring the topsheet 5 to have a predetermined fiber composition ratio, the distance between constituent fibers that constitute the topsheet 5 is increased by heat generated during use and air warmed by the heat. Accordingly, the volume of the sheet can be restored. As a result, the warming device 1 can exhibit high levels of flexibility and fit when in use.

In particular, in addition to configuring the topsheet 5 to have a predetermined fiber composition ratio, by using a configuration in which steam is generated in such a manner that the total amount of steam generated for 10 minutes is 10 mg/10 min or more, heat generated from the heat generating element and a predetermined amount or more of steam generated along with the heat generation function to more effectively restore the volume of the topsheet, and thus the warming device 1 can exhibit higher levels of flexibility and fit when in use.

The fibers contained in the topsheet 5 that is the portion that comes into contact with the target to be warmed may be present in any of the following configurations. Specific examples include: a configuration (i) in which the crimped fibers and the non-crimped fibers are present in a uniformly mixed manner in the portion that comes into contact with the target to be warmed; a configuration (ii) in which, when the portion that comes into contact with the target to be warmed is viewed in plan view, the portion is composed of a portion where only the crimped fibers are present and a portion where only the non-crimped fibers are present; a configuration (iii) in which the topsheet 5 has a multi-layer structure including a layer composed only of the crimped fibers and another layer composed only of the non-crimped fibers; and the like.

Among these configurations, from the viewpoint of more effectively exhibiting flexibility and a fit, it is more preferable that the topsheet 5 has a sheet configuration in which the crimped fibers and the non-crimped fibers are entangled with each other so as to form contacting parts where the crimped fibers and the non-crimped fibers are in contact with each other, and it is even more preferable that the topsheet 5 has the configuration (i) described above. In particular, in the case of the sheet configuration in which the fibers are entangled with each other, the topsheet 5 is preferably a non-woven fabric.

The crimped fibers contained in the topsheet 5 are two-dimensionally or three-dimensionally crimped fibers that have a crimp configuration such as a spiral configuration, a zigzag configuration, a U-shaped configuration, or a combination thereof. Examples of the crimped fibers include fibers with the crimp configuration described above obtained by heating latently crimpable fibers such as concentric core-sheath composite fibers, eccentric core-sheath composite fibers, or side-by-side composite fibers that are composed of two types of thermoplastic resins that have different shrinkage rates, and the like. That is, the latently crimpable fibers are fibers that are not crimped before being heated, but are crimped by being heated and form into crimped fibers.

Examples of thermoplastic resins include polyethylene (PE), polypropylene (PP), polyesters such as polyethylene terephthalate (PET) and polybutylene terephthalate (PBT), ethylene-a olefin copolymers such as an ethylene-propylene copolymer, polyamides, combinations thereof, and the like. Other examples include thermoplastic resins that are disclosed in JP H9-296325, and the like. As the combination of two types of thermoplastic resins that have different shrinkage rates, for example, in the case of core-sheath fibers, a combination of a high-melting point resin that is used as the core portion of the core-sheath fibers and a resin that has a melting point lower than that of the high-melting point resin and is used as the sheath portion of the core-sheath fibers may be used. In the case of side-by-side composite fibers, a combination of a first resin that has a high melting point and a second resin that has a melting point lower than that of the first resin may be used. Specifically, examples of the combination of the core and the sheath in the core-sheath fibers and the combination of the first resin and the second resin in the side-by-side composite fibers include fibers composed of combinations of a polypropylene/ethylene-propylene copolymer, polyethylene terephthalate/polyethylene, polyethylene terephthalate/polypropylene, and the like. These may be used alone or in a combination of two or more.

Whether or not the fibers that constitute the topsheet 5 are crimped fibers can be determined based on, for example, the crimp ratio and the number of crimps specified by JIS L 0208. Where a length of a measurement target fiber measured when the fiber is stretched out is represented by length W1, and a length of the measurement target fiber in a natural state is represented by length W2, the crimp ratio is defined as a percentage of the difference between the length W1 and the length W2 relative to the length W1, and calculated from an equation represented by 100 × (W1 - W2) / W1 (%). The length W2 refers to a length between opposite end portions of the fiber in a natural state when connected with a straight line. The natural state refers to a state in which the fiber extends downward due to its own weight, with one end portion of the fiber being fixed to a horizontal plate. The length W1 of the fiber when the fiber is stretched out refers to a length at the minimum load when the fiber is stretched out until there is no crimp.

The crimped fibers that constitute the topsheet 5 have a crimp ratio specified by JIS L 0208 of preferably 5% or more, more preferably 10% or more, even more preferably 15% or more, and yet even more preferably 20% or more. Also, the crimp ratio of the crimped fibers is preferably 80% or less, more preferably 70% or less, even more preferably 60% or less, and yet even more preferably 50% or less.

In the crimped fibers that constitute the topsheet 5, the number of crimps per cm of fiber length specified by JIS L 0208 is preferably 1 or more, more preferably 30 or more, even more preferably 50 or more, and yet even more preferably 80 or more. The number of crimps in the crimped fibers is preferably 200 or less, more preferably 180 or less, even more preferably 150 or less, and yet even more preferably 120 or less. The crimp ratio and the number of crimps within the described-above ranges can be obtained by, for example, adjusting, as appropriate, crimping processing performed in the fiber production process, the material of the latently crimpable fibers, the temperature of heating processing, and the like.

From the viewpoint of improving the fit when the warming device 1 is in use, the mass ratio of the crimped fibers relative to the total mass of the crimped fibers and the non-crimped fibers in the topsheet 5 is preferably 50 mass% or less, more preferably 45 mass% or less, even more preferably 40 mass% or less, and yet even more preferably 35 mass% or less. Also, from the viewpoint of achieving a good texture when the warming device 1 is in use, the mass ratio of the crimped fibers relative to the total mass of the crimped fibers and the non-crimped fibers is preferably 5 mass% or more, more preferably 10 mass% or more, even more preferably 15 mass% or more, and yet even more preferably 20 mass% or more.

Also, from the viewpoint of improving the fit when the warming device 1 is in use, the mass ratio of the non-crimped fibers relative to the total mass of the crimped fibers and the non-crimped fibers in the topsheet 5 is preferably 50 mass% or more, more preferably 55 mass% or more, even more preferably 60 mass% or more, and yet even more preferably 65 mass% or more. Also, from the viewpoint of achieving a good texture when the warming device 1 is in use, the mass ratio of the non-crimped fibers relative to the total mass of the crimped fibers and the non-crimped fibers is preferably 95 mass% or less, more preferably 90 mass% or less, even more preferably 85 mass% or less, yet even more preferably 80 mass% or less, and yet even more preferably 75 mass% or less.

The ratio of the crimped fibers or the non-crimped fibers that constitute the topsheet 5 can be obtained in the following manner. For example, ten fibers are randomly extracted from a fibrous sheet that is a measurement target. Fibers that satisfy a condition in which the crimp ratio measured according to JIS L 0208 is 5% or more and a condition in which the number of crimps per cm of fiber length is 1 or more are determined as "crimped fibers", and fibers that do not satisfy either one of the condition in which the crimp ratio is 5% or more and the condition in which the number of crimps per cm of fiber length is 1 or more are determined as "non-crimped fibers". The ratio of the crimped fibers or the non-crimped fibers can be calculated as the mass ratio relative to the total mass of the crimped fibers and the non-crimped fibers.

As described above, the non-crimped fibers contained in the topsheet 5 refer to fibers that do not satisfy either one of the condition in which the crimp ratio is 5% or more and the condition in which the number of crimps per cm of fiber length is 1 or more. Examples of the non-crimped fibers include: synthetic fibers that contain one or more of the thermoplastic resins described above; natural fibers such as wood pulp fibers, cotton fibers, and hemp fibers; regenerated fibers such as rayon and cupra; and the like. These may be used alone or in a combination of two or more.

In the present embodiment, from the viewpoint of reducing the number of contacting parts between the crimped fibers and the non-crimped fibers and efficiently obtaining a voluminous topsheet, it is preferable that the non-crimped fibers that constitute the topsheet 5 have a fineness smaller than that of the crimped fibers that constitute the topsheet 5.

More specifically, the fineness of the crimped fibers is preferably 1.0 dtex or more, more preferably 1.2 dtex or more, even more preferably 1.3 dtex or more, and yet even more preferably 1.6 dtex or more. Also, the fineness of the crimped fibers is preferably 3.0 dtex or less, more preferably 2.7 dtex or less, even more preferably 2.5 dtex or less, and yet even more preferably 2.2 dtex or less. In the case where two or more types of crimped fibers are used, an arithmetic mean value obtained by weighting the fineness of each type of crimped fibers that constitute the topsheet 5 according to the mass ratio of the crimped fibers is used as the fineness of the crimped fibers.

The non-crimped fibers have a fineness of preferably 0.5 dtex or more, more preferably 0.8 dtex or more, and even more preferably 1.0 dtex or more under the condition that the non-crimped fibers have a fineness smaller than that of the crimped fibers. Also, the non-crimped fibers have a fineness of preferably 2.0 dtex or less, more preferably 1.5 dtex or less, and even more preferably 1.2 dtex or less. As a result of each of the crimped fibers and the non-crimped fibers having a fineness within the above-described range, the topsheet 5 exhibits an excellent level of flexibility and a good texture when touched, and the warming device 1 provides an improved fit. Accordingly, the user feels better during use. In the case where two or more types of non-crimped fibers are used, an arithmetic mean value obtained by weighting the fineness of each type of non-crimped fibers that constitute the topsheet 5 according to the mass ratio of the non-crimped fiber is used as the fineness of the non-crimped fibers.

Also, as another embodiment of the topsheet 5 of the warming device 1, it is preferable that the topsheet is configured to increase the thickness thereof within a predetermined range due to heating. In particular, it is preferable that the topsheet is configured to increase the thickness thereof within a predetermined range after a period of 1 hour after the start of use as well as at the end of heat generation, or is maintained at the thickness to keep the volume of the topsheet 5. With this amount of increase in thickness, the volume of the topsheet 5 can be further increased. Accordingly, a better flexibility and texture when touched can be achieved, and a fit to the target to be warmed can be improved. The amount of increase in thickness may be satisfied instead of or in addition to the configurations of the above-described embodiments. In the present embodiment as well, the topsheet 5 is preferably an air permeable fibrous sheet. The following description will be given focusing mainly on structural elements that are different from those of the embodiments given above. Accordingly, the same structural elements are given the same reference numerals, and a description thereof will be omitted. For structural elements that are not specifically described below, see the description of the warming device given above as appropriate.

The following description will be given using an unused warming device 1 hermetically housed in a package or the like. The time required from when the package is opened to when the topsheet 5 of the warming device 1 is placed spread on a flat surface while exposing the same to an oxygen-containing atmosphere such as the air, or in other words, the time after seconds have passed after opening the package is defined as "the start of heat generation", and the time after 24 hours have passed after the start of heat generation is defined as "the end of the heat generation". At this time, the thickness of the topsheet 5 of the present embodiment preferably has increased or is maintained.

From the viewpoint of achieving excellent levels of flexibility and fit, the amount of increase Ta in thickness of the topsheet 5 during a period from the start of heat generation to the end of the heat generation is preferably 0.20 mm or more, more preferably 0.30 mm or more, even more preferably 0.40 mm or more, and yet even more preferably 0.45 mm or more. Also, from the viewpoint of suppressing excessive compression on the target to be warmed, the amount of increase Ta in thickness of the topsheet 5 during the period is preferably 0.70 mm or less, more preferably 0.65 mm or less, even more preferably 0.6 mm or less, and yet even more preferably 0.55 mm or less. With this configuration, the volume of the topsheet 5 recovers as a result of the warming device 1 generating heat, and thus the flexibility of the topsheet 5 can be improved during a period from the start to the end of use, and an excellent level of fit can be provided. Furthermore, the user is unlikely to feel discomfort, such as excessive compression, caused as a result of the volume of the topsheet 5 increasing, and both flexibility and texture when touched can be improved. The amount of increase Ta in thickness can be obtained by subtracting a sheet thickness T1 (mm) measured at the start of heat generation from a sheet thickness T4 (mm) measured at the end of the heat generation. The method for measuring the sheet thickness T1 and the sheet thickness T4 will be described later.

Also, from the viewpoint of causing the warming device to exhibit both flexibility and a fit at an early stage after the warming device is worn, the amount of increase Tc in thickness of the topsheet 5 measured 5 minutes after the start of heat generation is preferably 0.10 mm or more, more preferably 0.20 mm or more, even more preferably 0.30 mm or more, and yet even more preferably 0.35 mm or more. Also, from the viewpoint of suppressing excessive compression on the target to be warmed, the amount of increase in thickness of the topsheet 5 during the period is preferably 0.60 mm or less, more preferably 0.55 mm or less, even more preferably 0.50 mm or less, and yet even more preferably 0.45 mm or less. As a result of achieving the amount of increase in thickness as described above, even when the warming device 1 is worn immediately after being taken out from the package or the like, the thickness of the topsheet 5 increases in a short period of time and becomes voluminous. Accordingly, the fit to the target to be warmed can be sufficiently enhanced. Furthermore, the user is unlikely to feel discomfort, such as excessive compression, caused as a result of the volume of the topsheet 5 increasing, and both flexibility and texture when touched can be improved. The amount of increase Tc in thickness can be obtained by subtracting the sheet thickness T1 (mm) measured at the start of heat generation from a sheet thickness T2 (mm) measured 5 minutes after the start of heat generation. The method for measuring the sheet thickness T2 will be described later.

Also, likewise, from the viewpoint of achieving excellent levels of flexibility and fit for a long period of time, the amount of increase Tb in thickness of the topsheet 5 during a period of 1 hour after the start of heat generation is preferably 0.15 mm, more preferably 0.20 mm or more, even more preferably 0.30 mm or more, and yet even more preferably 0.40 mm or more. Also, from the viewpoint of suppressing excessive compression on the target to be warmed, the amount of increase in thickness of the topsheet 5 during the period is preferably 0.70 mm or less, more preferably 0.65 mm or less, and even more preferably 0.60 mm or less. With this configuration, the volume of the topsheet 5 recovers as a result of the warming device 1 generating heat, and thus the flexibility of the topsheet 5 can be improved for a long period of time from the start of use, and an excellent level of fit can be obtained. In particular, the period of 1 hour after the start of heat generation serves as an indicator for continuous use of the warming device 1. Accordingly, when the amount of increase Tb in thickness is within the above-described range, it is possible to provide an advantage in that the wearer of the warming device 1 can sufficiently perceive the flexibility of the topsheet 5 and the fit of the warming device 1. In addition thereto, the wearer is unlikely to feel discomfort, such as excessive compression, caused as a result of the volume of the topsheet 5 increasing, and both flexibility and texture when touched can be improved. The amount of increase Tb in thickness can be obtained by subtracting the sheet thickness T1 (mm) measured at the start of heat generation from a sheet thickness T3 (mm) measured 1 hour after the start of heat generation. The method for measuring the sheet thickness T3 will be described later.

Also, from the viewpoint of ensuring an appropriate thickness at the time after 5 minutes have passed from the start of heat generation, at the time after 1 hour has passed from the start of heat generation, and the end of heat generation, the thickness T1 of the topsheet 5 of the warming device 1 at the start of heat generation is preferably 0.8 mm or more, more preferably 1.0 mm or more, even more preferably 1.1 mm or more, and yet even more preferably 1.2 mm or more. Also, from the viewpoint of suppressing compression caused by the thickness excessively increasing at the time after 5 minutes have passed from the start of heat generation, at the time after 1 hour has passed from the start of heat generation, the thickness T1 of the topsheet 5 of the warming device 1 at the start of heat generation is preferably 3.0 mm or less, more preferably 2.5 mm or less, even more preferably 2.0 mm, and yet even more preferably 1.5 mm or less.

The amount of increase in thickness of the topsheet 5 can be measured using, for example, the following method. First, the sheet thickness of the topsheet 5 at the start of heat generation is measured at three or more locations in the topsheet 5 using a constant pressure thickness gauge or the like by applying a load of 3.7 gf/cm² to the topsheet 5, and the arithmetic mean value of the measured thickness values is defined as the sheet thickness T1 (mm) measured at the start of heat generation. Likewise, the sheet thickness of the topsheet 5 at the end of the heat generation is measured in the same manner under the same load, and the arithmetic mean value of the measured thickness values is defined as the sheet thickness T4 (mm) measured at the end of the heat generation. Likewise, the sheet thickness T2 (mm) measured 5 minutes after the start of heat generation and the sheet thickness T3 (mm) measured 1 hour after the start of heat generation can be obtained and calculated under the same load using the same method as described above. The amount of increase Ta in thickness can be obtained based on an equation represented by T4 - T1 (mm), the amount of increase Tb in thickness can be obtained based on an equation represented by T3 - T1 (mm), and the amount of increase Tc in thickness can be obtained based on an equation represented by T2 - T1 (mm). The method for measuring the amount of increase in thickness of the topsheet 5 will be described in detail in Examples given later.

Also, as yet another embodiment of the topsheet 5 of the warming device 1, it is preferable that the topsheet 5 is an air permeable fibrous sheet that contains two or more types of materials that have different melting points. With this configuration, it is possible to sufficiently apply warm heat to the target to be warmed such as the eyes and the vicinity of the eyes, and at the same time, as a result of the volume of the topsheet 5 of the warming device 1 increasing, both flexibility and fit of the topsheet 5 can be improved to excellent levels. The configuration that the topsheet 5 contains two or more types of fibers may be satisfied instead of or in addition to the configurations of the above-described embodiments. The following description will also be given focusing mainly on structural elements that are different from those of the embodiments given above. Accordingly, the same structural elements are given the same reference numerals, and a description thereof will be omitted. For structural elements that are not specifically described below, see the description of the warming device given above as appropriate.

As the material that constitutes the topsheet 5, for example, at least one or more of synthetic fibers made of one or more of the thermoplastic resins described above, the above-described natural fibers, the above-described regenerated fibers, and the like can be used. In the case where fibers that contain materials that have different melting points are used as the material that constitutes the topsheet 5, examples of combinations of the materials include: (a) a configuration in which natural fibers are used as one type of fibers, and synthetic fibers are used as another type of fibers; (b) a configuration in which two or more types of materials are contained in each fiber, such as natural fibers or regenerated fibers whose surface being covered with a thermoplastic resin, or fibers, such as core-sheath fibers or side-by-side fibers, that contain two or more types of thermoplastic resins; (c) a configuration in which the topsheet 5 is made using two or more types of fibers that have different melting points and these fibers are both natural fibers; (d) a configuration in which the topsheet 5 is made using two or more types of fibers that have different melting points and these fibers are both synthetic fibers; and the like.

In the case of the configuration in which the topsheet 5 is made using two or more types of fibers that have different melting points and these fibers are both synthetic fibers, one type of synthetic fibers may be fibers composed only of one type of thermoplastic resin, and another type of synthetic fibers may be fibers composed of two or more types of thermoplastic resins. Alternatively, two types of synthetic fibers both may be fibers composed of two or more types of thermoplastic resins. In the case where fibers composed only of one type of raw material are used, the melting point of the fibers is the same as the melting point of the raw material. In the case where fibers composed of two or more different types of raw materials are used, the melting point of the fibers may be different from the melting point of one of the raw materials that constitute one type of fibers and the melting point of one of the raw materials that constitute another type of fibers. In the case where it is not possible to measure the melting point due to carbonization or the like, differential scanning calorimetry (DSC) measurement is performed, and the peak temperature is defined as the melting point. In particular, when a fibrous sheet that contains two or more types of synthetic fibers that have different melting points is subjected to heating processing, due to the difference in melting point, the fibers that contain a low-melting point resin are likely to be crimped. Accordingly, it is possible to obtain an advantage in that a fibrous sheet that contains crimped fibers and non-crimped fibers can be easily produced as a topsheet 5.

At least one type of fibers of the constituent fibers that constitute the topsheet 5 preferably include a thermoplastic resin, more preferably include one or more selected from the group consisting of polypropylene, polyethylene, polyethylene terephthalate, and an ethylene-a olefin copolymer, even more preferably include at least polypropylene, and yet even more preferably include synthetic fibers composed of polypropylene and an ethylene-a olefin copolymer. With this configuration, the flexibility and fit of the topsheet 5 can be efficiently exhibited, and a sufficient strength can be exhibited when the warming device 1 is in use. In particular, it is advantageous to use fibers that contain two or more types of thermoplastic resins because the fibers can be crimped. Also, in the case where two types of fibers are used as the materials that have different melting points, from the viewpoint of obtaining a very voluminous topsheet, it is preferable to use a combination of, for example, first fibers composed of polypropylene and an ethylene-propylene copolymer and second fibers composed of polypropylene.

The mass ratio of thermoplastic resin relative to the total mass of the constituent fibers that constitute the topsheet 5 is preferably 10 mass% or more, more preferably 30 mass% or more, even more preferably 50 mass% or more, and yet even more preferably 100 mass%. By adjusting the mass ratio of thermoplastic resin within the above-described range, the flexibility and fit of the warming device achieved by the topsheet 5 can be increased, and at the same time, various bonding methods such as heat and an adhesive can be used when bonding the topsheet 5 to another constituent member. Accordingly an advantage of improving the productivity of the warming device can be obtained. The higher the mass ratio of thermoplastic resin, the more advantageous it is to improve the productivity of the warming device. The mass of thermoplastic resin can be obtained by performing, for example, differential scanning calorimetry (DSC) measurement on the topsheet 5 that is a measurement target, and defining a value calculated from the peak area of the resin as a mass W1 of thermoplastic resin. In the case where the topsheet 5 contains a plurality of thermoplastic resins, the mass of each resin is calculated from the peak area of the resin, and the sum of the calculated masses is defined as the mass W1 of thermoplastic resin. The mass ratio of thermoplastic resin can be determined as a percentage of the mass W1 relative to a mass W2 of the topsheet.

Hereinafter, matters that are applied to the embodiments in common will be described. A sheet material that can be used as the heat generating elements 3, the ear loop portions 4, the topsheet 5, or the backsheet 6 may be determined as appropriate by taking into consideration the air permeability, moisture permeability, texture, elasticity, and strength of the member, as well as properties such as preventing leakage of materials that constitute the heat generation sheet and the heat generation composition. For example, a non-woven fabric, a woven fabric, a fibrous sheet such as paper, a resin foamed sheet, a metal sheet, or a combination thereof can be used.

As a sheet material that has a high air permeability, a melt-blown non-woven fabric is preferably used. As a sheet material that can be used to achieve a good texture, an air-through non-woven fabric and a thermal bonded non-woven fabric are preferably used. As a sheet material that can be used to exhibit elasticity, for example, an air-through non-woven fabric, a spun bonded non-woven fabric, a thermal bonded non-woven fabric, and the like that contain synthetic fibers made of polyester such as polyethylene terephthalate, polyethylene, or polypropylene. As a sheet material that can be used to apply strength, a spun bonded non-woven fabric, a spun laced non-woven fabric, a needle punched non-woven fabric, a chemically bonded non-woven fabric, and the like are preferably used. In addition to or instead of the above-described non-woven fabrics, it is also possible to use non-woven fabrics whose surface has been treated with silicone, a surfactant or the like, foamed sheets made of thermoplastic resin such as polyethylene or polyurethane, and the like. Also, these sheet materials may be made using a combination of a plurality of fibers that are made of different fiber materials and have different fiber diameters and different degrees of crimping, or a plurality of sheet materials may be combined to exhibit desired properties. The heat generating elements 3, the ear loop portions 4, the topsheet 5, and the backsheet 6 each may have a single structure composed only of a single sheet material including one or more layers, or a stack structure in which two or more types of sheet materials are stacked.

As described above, it is preferable to use a fibrous sheet as the topsheet 5. From the viewpoint of easily obtaining a fibrous sheet that contains a thermoplastic resin, at least one of a needle punched non-woven fabric, an air-through non-woven fabric, a spun bonded non-woven fabric, and a chemically bonded non-woven fabric can be preferably used. In the case where the topsheet 5 has a stack structure in which sheet materials are stacked, and each sheet material contains crimped fibers, the sheet materials each independently satisfy the crimp ratio and the number of crimps described above.

In the case where an air permeable fibrous sheet is used as the topsheet 5, the air permeability of the topsheet 5 is preferably 0.01 sec/100 mL or more, more preferably 50 sec/100 mL or more, and even more preferably 2000 sec/100 mL or more. Also, the air permeability of the topsheet 5 is preferably 15000 sec/100 mL or less, more preferably 50000 sec/100 mL or less, and even more preferably 10000 sec/100 mL or less. The air permeability is measured using the method according to JIS P8117. When the air permeability takes a small value, it means that it does not take time for the air to pass through the topsheet 5, and thus the air permeability is high.

In the case where a non-woven fabric is used as the backsheet 6, it is preferable to use a fibrous sheet that has an air permeability higher than that of the topsheet 5. That is, the backsheet 6 preferably has an air permeability measured using the method according to JIS P8117 higher than that of the topsheet 5. More specifically, the higher the air permeability of the backsheet 6, the more preferable it is. Under the condition that the backsheet 6 has an air permeability higher than that of the topsheet 5, the air permeability of the backsheet 6 is preferably 50 sec/100 mL or more, more preferably 4000 sec/100 mL or more, and even more preferably 20000 sec/100 mL or more, and it is more preferable that the backsheet 6 is a non-air permeable sheet.

In the case where non-woven fabrics are used as the topsheet 5 and the backsheet 6, the basis weight of the topsheet 5 is preferably 10 g/m² or more, and more preferably 20 g/m² or more. The basis weight of the topsheet 5 is preferably 200 g/m² or less, and more preferably 130 g/m² or less.

Also, from the viewpoint of improving heat retention properties of the application area during use, the basis weight of the backsheet 6 is preferably higher than that of the topsheet 5. More specifically, the basis weight of the backsheet 6 is preferably 10 g/m² or more, and more preferably 30 g/m² or more. The basis weight of the backsheet 6 is preferably 150 g/m² or less, and more preferably 200 g/m² or less. In the case where the topsheet 5 and the backsheet 6 each have a stack structure, it is sufficient that the basis weight of the entire sheet is within the above-described range.

The flexural rigidity of the warming device 1 in the lateral direction X is preferably 0.80 N or more, more preferably 1.00 N or more, even more preferably 1.01 N or more. Also, the flexural rigidity of the warming device 1 in the lateral direction X is preferably 1.20 N or less, more preferably 1.10 N or less, even more preferably 1.06 N or less, and yet even more preferably 1.03 N or less. As a result of having a flexural rigidity within the above-described range, the warming device 1 provides a high level of flexibility in the lateral direction X and a further improved fit when the warming device 1 is worn. The method for measuring flexural rigidity will be described in detail in Examples given later.

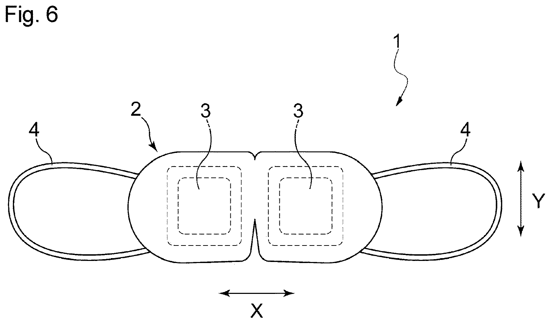

The configuration of the ear loop portions 4 in the warming device 1 is not limited to the sheet-like members shown in Figs. 1 and 2 as long as the enclosing material 2 can be fixed to the eyes of the user. For example, as shown in Fig. 6, ear loop portions 4 made using strap-like members or ear loop portions 4 made using thread-like or band-like members may be used. From the viewpoint of enhancing the fit of the warming device, the ear loop portions 4 are preferably made using an elastic body such as a rubber and stretchable.

In the description given above, as the configuration of the heat generating element 3 of the warming device 1, a configuration has been described in which two heat generating elements 3 are held spaced apart from each other. However, there is no particular limitation on the configuration of the warming device as long as it is possible to apply warmth to the eyes of the user and the vicinity thereof. For example, one heat generating element that has a shape and a size that can cover the eyes of the user and the vicinity thereof may be held between the topsheet 5 and the backsheet 6, or three or more heat generating elements may be held between the topsheet 5 and the backsheet 6.

Also, the heat generating elements 3 shown in Figs. 2 and 3 are configured in such a manner that a portion thereof is fixed only to a center region of the warming device 1 in the lateral direction X, but the configuration is not limited thereto. For example, the heat generating elements 3 and the backsheet 6 may be continuously or intermittently bonded in the center region and a region other than the center region using an adhesive, or the heat generating elements 3 may be bonded to the backsheet 6 by applying an adhesive to the entire surface of portions of the backsheet 6 where the heat generating elements 3 are disposed.

In the warming device according to the present disclosure, any one of the structural elements described above may be used alone, two or more of the structural elements described above may be combined, or all of the structural elements described above may be combined. Specifically, the fibrous sheet that comes into contact with the target to be warmed when the warming device 1 is in use may satisfy any one of the following configurations: (i) the ratio of crimped fibers and non-crimped fibers; (ii) the amount of increase in thickness of the sheet during a period from the start of heat generation to the end of the heat generation; (iii) the amount of increase in thickness of the sheet measured 5 minutes after the start of heat generation; (iv) the amount of increase in thickness of the sheet measured 1 hour after the start of heat generation; (v) the fineness of constituent fibers of the sheet; and (vi) the amount of resin contained in the constituent fibers of the sheet, or may satisfy any two of the above-described configurations (i) to (vi), any three of the above-described configurations (i) to (vi), any four of the above-described configurations (i) to (vi), any five of the above-described configurations (i) to (vi), or all of the above-described configurations (i) to (vi). Configurations other than the above-described configurations (i) to (vi) of the present disclosure can also be used alone or in an appropriate combination.

The present invention has been described above based on preferred embodiments thereof, but the present invention is not limited to the embodiment.

With respect to the embodiments of the present invention described above, warming devices as described below are also disclosed.

<1> A warming device comprising:
   a heat generating element; and
   an enclosing material that houses the heat generating element,
   wherein a portion of the enclosing material that comes into contact with a target to be warmed when the warming device is in use comprises an air permeable fibrous sheet that contains crimped fibers and non-crimped fibers,
   a ratio of the non-crimped fibers relative to a total of the crimped fibers and the non-crimped fibers is 50 mass% or more,
   the heat generating element contains an oxidizable metal powder, a carbon material powder and water, and
   the heat generating element has a function of generating steam along with heat generation.
<2> The warming device as set forth in clause <1>,
   wherein the ratio of the non-crimped fibers relative to the total of the crimped fibers and the non-crimped fibers is more preferably 55 mass% or more, even more preferably 60 mass% or more, and yet even more preferably 65 mass% or more.
<3> The warming device as set forth in clause <1> or <2>,
   wherein the ratio of the non-crimped fibers relative to the total of the crimped fibers and the non-crimped fibers is preferably 95 mass% or less, more preferably 90 mass% or less, even more preferably 85 mass% or less, yet even more preferably 80 mass% or less, and yet even more preferably 75 mass% or less.
<4> The warming device as set forth in any one of clauses <1> to <3>,
   wherein the ratio of the crimped fibers relative to the total of the crimped fibers and the non-crimped fibers is more preferably 45 mass% or less, even more preferably 40 mass% or less, and yet even more preferably 35 mass% or less.
<5> The warming device as set forth in any one of clauses <1> to <4>,
   wherein the ratio of the crimped fibers relative to the total of the crimped fibers and the non-crimped fibers is preferably 5 mass% or more, more preferably 10 mass% or more, even more preferably 15 mass% or more, and yet even more preferably 20 mass% or more.
<6> The warming device as set forth in any one of clauses <1> to <5>,
   wherein the non-crimped fibers have a fineness smaller than that of the crimped fibers.
<7> The warming device as set forth in any one of clauses <1> to <6>,
   wherein the crimped fibers have a fineness of 1.0 dtex or more and 3.0 dtex or less.
<8> The warming device as set forth in any one of clauses <1> to <7>,
   wherein the crimped fibers have a fineness of more preferably 1.2 dtex or more, even more preferably 1.3 dtex or more, and yet even more preferably 1.6 dtex or more.
<9> The warming device as set forth in any one of clauses <1> to <8>,
   wherein the crimped fibers have a fineness of more preferably 2.7 dtex or less, even more preferably 2.5 dtex or less, and yet even more preferably 2.2 dtex or less.
<10> The warming device as set forth in any one of clauses <1> to <9>,
   wherein the non-crimped fibers have a fineness of preferably 0.5 dtex or more, more preferably 0.8 dtex or more, and even more preferably 1.0 dtex or more.
<11> The warming device as set forth in any one of clauses <1> to <10>,
   wherein the non-crimped fibers have a fineness of preferably 2.0 dtex or less, more preferably 1.5 dtex or less, and even more preferably 1.2 dtex or less.
<12> The warming device as set forth in any one of clauses <1> to <11>,
   wherein the fibrous sheet maintains a sheet configuration in which the crimped fibers and the non-crimped fibers are entangled with each other, and
   the fibrous sheet includes contacting parts where the crimped fibers and the non-crimped fibers are in contact with each other.
<13> The warming device as set forth in any one of clauses <1> to <12>,
   wherein the portion of the fibrous sheet that comes into contact with the target to be warmed is configured in such a manner that the crimped fibers and the non-crimped fibers are present in a uniformly mixed manner.
<14> The warming device as set forth in any one of clauses <1> to <13>,
   wherein the crimped fibers are concentric core-sheath composite fibers, eccentric core-sheath composite fibers, or side-by-side composite fibers that comprise two different types of thermoplastic resins that have different shrinkage rates, and
   a combination of the thermoplastic resins is one or more of the following combinations: a combination of polypropylene and an ethylene-propylene copolymer; a combination of polyethylene terephthalate and polyethylene; and a combination of polyethylene terephthalate and polypropylene.
<15> The warming device as set forth in any one of clauses <1> to <14>,
   wherein the crimped fibers have a crimp ratio of preferably 5% or more, more preferably 10% or more, even more preferably 15% or more, and yet even more preferably 20% or more,
   the crimp ratio is preferably 80% or less, more preferably 70% or less, even more preferably 60% or less, and yet even more preferably 50% or less, and
   the crimp ratio is specified by JIS L 0208.
<16> The warming device as set forth in any one of clauses <1> to <15>,
   wherein the crimped fibers have a number of crimps per cm of fiber length of preferably 1 or more, more preferably 30 or more, even more preferably 50 or more, and yet even more preferably 80 or more,
   the number of crimps is preferably 200 or less, more preferably 180 or less, even more preferably 150 or less, and yet even more preferably 120 or less, and
   the number of crimps is specified by JIS L 0208.
<17> A warming device comprising:
   a heat generating element; and
   an enclosing material that houses the heat generating element,

   wherein a portion of the enclosing material that comes into contact with a target to be warmed when the warming device is in use comprises a fibrous sheet,
   the fibrous sheet is configured to increase the thickness thereof by 0.20 mm or more and 0.70 mm or less during a period from a start to an end of heat generation by the warming device,
   the heat generating element contains an oxidizable metal powder, a carbon material powder and water, and
   the heat generating element has a function of generating steam along with heat generation.
<18> The warming device as set forth in clause <17>,
   wherein the fibrous sheet is configured to increase the thickness thereof by more preferably 0.30 mm or more, even more preferably 0.40 mm or more, and yet even more preferably 0.45 mm or more, and more preferably 0.65 mm or less, even more preferably 0.6 mm or less, and yet even more preferably 0.55 mm or less during the period from the start to the end of heat generation by the warming device.
<19> A warming device comprising:
   a heat generating element; and
   an enclosing material that houses the heat generating element,

   wherein a portion of the enclosing material that comes into contact with a target to be warmed when the warming device is in use comprises a fibrous sheet,
   the fibrous sheet is configured to increase the thickness thereof by 0.10 mm or more and 0.60 mm or less during a period of 5 minutes after a start of heat generation by the warming device,
   the heat generating element contains an oxidizable metal powder, a carbon material powder and water, and
   the heat generating element has a function of generating steam along with heat generation.
<20> The warming device as set forth in any one of clauses <17> to <19>,
   wherein the fibrous sheet is configured to increase the thickness thereof by more preferably 0.20 mm or more, even more preferably 0.30 mm or more, and yet even more preferably 0.35 mm or more, and more preferably 0.55 mm or less, even more preferably 0.50 mm or less, and yet even more preferably 0.45 mm or less during a period of 5 minutes after the start of heat generation by the warming device.
<21> A warming device comprising:
   a heat generating element; and
   an enclosing material that houses the heat generating element,

   wherein a portion of the enclosing material that comes into contact with a target to be warmed when the warming device is in use comprises a fibrous sheet,
   the fibrous sheet is configured to increase the thickness thereof by 0.15 mm or more and 0.70 mm or less during a period of 1 hour after a start of heat generation by the warming device,
   the heat generating element contains an oxidizable metal powder, a carbon material powder and water, and
   the heat generating element has a function of generating steam along with heat generation.
<22> The warming device as set forth in any one of clauses <17> to <21>,
   wherein the fibrous sheet is configured to increase the thickness thereof by more preferably 0.20 mm or more, even more preferably 0.30 mm or more, and yet even more preferably 0.40 mm or more, and more preferably 0.65 mm or less, and even more preferably 0.60 mm or less during a period of 1 hour after the start of heat generation by the warming device.
<23> The warming device as set forth in any one of clauses <17> to <22>,
   wherein the thickness of the fibrous sheet at the start of heat generation is preferably 0.8 mm or more, more preferably 1.0 mm or more, even more preferably 1.1 mm or more, and yet even more preferably 1.2 mm or more, and preferably 3.0 mm or less, more preferably 2.5 mm or less, even more preferably 2.0 mm, and yet even more preferably 1.5 mm or less.
<24> A warming device comprising:
   a heat generating element; and
   an enclosing material that houses the heat generating element,
   wherein a portion of the enclosing material that comes into contact with a target to be warmed when the warming device is in use comprises an air permeable fibrous sheet that contains two or more of materials that have different melting points.
<25> The warming device as set forth in clause <24>,
   wherein at least one of the materials contains a thermoplastic resin.
<26> The warming device as set forth in clause <25>,
   wherein the thermoplastic resin includes one or more selected from the group consisting of polypropylene, polyethylene, and polyethylene terephthalate.
<27> The warming device as set forth in any one of clauses <24> to <26>,
   wherein the fibrous sheet contains fibers comprising polypropylene.
<28> The warming device as set forth in any one of clauses <24> to <27>, wherein the fibrous sheet contains:
   first fibers comprising polypropylene and an ethylene-propylene copolymer; and
   second fibers comprising polypropylene.
<29> The warming device as set forth in any one of clauses <1> to <28>,
   wherein the heat generating element has a function of generating steam along with heat generation.
<30> The warming device as set forth in any one of clauses <1> to <29>,
   wherein the warming device includes:
      the enclosing material that is shaped to cover user's eyes when the warming device is in use;
      the heat generating element that is housed in the enclosing material; and
      a pair of ear loop portions that are attached to the enclosing material and can
      keep the user's eyes covered with the enclosing material,
   wherein the enclosing material includes:
      the fibrous sheet that is located on a side that is close to user's skin; and
      a backsheet that is located on a side that is far from the user's skin, and the heat generating element is held between the fibrous sheet and the backsheet.
<31> The warming device as set forth in clause <30>,
   wherein the backsheet comprises a non-woven fabric.
<32> The warming device as set forth in clause <30> or <31>,
   wherein the backsheet has an air permeability higher than that of the fibrous sheet, the air permeability being measured according to a method according to JIS P8117.
<33> The warming device as set forth in any one of clauses <30> to <32>,
   wherein the backsheet has an air permeability of preferably 50 sec/100 mL or more, more preferably 4000 sec/100 mL or more, and even more preferably 20000 sec/100 mL or more, the air permeability being measured using a method according to JIS P8117, and it is more preferable that the backsheet is a non-air permeable sheet.
<34> The warming device as set forth in any one of clauses <30> to <33>,
   wherein the backsheet has a basis weight higher than that of the fibrous sheet.
<35> The warming device as set forth in any one of clauses <30> to <34>,
   wherein the backsheet has a basis weight of preferably 10 g/m² or more, and more preferably 30 g/m² or more, and
   the backsheet has a basis weight of preferably 150 g/m² or less, and more preferably 200 g/m² or less.
<36> The warming device as set forth in any one of clauses <30> to <35>,
   wherein the ear loop portions are preferably made using an elastic sheet material.
<37> The warming device as set forth in any one of clauses <30> to <36>,
   wherein the ear loop portions preferably comprise elastic strap-like members.
<38> The warming device as set forth in any one of clauses <30> to <37>,
   wherein the heat generating element includes a heat generation portion that contains an oxidizable metal, a reaction accelerator, an electrolyte and water.
<39> The warming device as set forth in any one of clauses <30> to <38>,
   wherein the heat generation portion comprises a fibrous sheet that contains an oxidizable metal, a reaction accelerator, fibrous material, an electrolyte and water.
<40> The warming device as set forth in any one of clauses <30> to <38>,
   wherein the heat generation portion comprises a heat generation composition in the form of a paste that contains an oxidizable metal, a reaction accelerator, a liquid retainer, an electrolyte and water.
<41> The warming device as set forth in any one of clauses <30> to <38>,
   wherein the heat generation portion comprises a heat generation composition in the form of a powder that contains an oxidizable metal, a reaction accelerator, a liquid retainer, an electrolyte and water.
<42> The warming device as set forth in any one of clauses <30> to <41>,
   wherein the heat generating element includes the heat generation portion and a pouch-like member that houses the heat generation portion.
<43> The warming device as set forth in any one of clauses <1> to <42>,
   wherein the fibrous sheet preferably comprises a non-woven fabric.
<44> The warming device as set forth in any one of clauses <1> to <43>,
   wherein the fibrous sheet is preferably at least one of a needle punched non-woven fabric, an air-through non-woven fabric, a spun bonded non-woven fabric, and a chemically bonded non-woven fabric.
<45> The warming device as set forth in any one of clauses <1> to <44>,
   wherein the fibrous sheet has an air permeability of preferably 0.01 sec/100 mL or more, more preferably 50 sec/100 mL or more, and even more preferably 2000 sec/100 mL or more,
   the air permeability is preferably 15000 sec/100 mL or less, more preferably 50000 sec/100 mL or less, and even more preferably 10000 sec/100 mL or less, and
   the air permeability being measured using a method according to JIS P8117.
<46> The warming device as set forth in any one of clauses <1> to <45>,
   wherein the fibrous sheet has a basis weight of preferably 10 g/m² or more, and more preferably 20 g/m² or more, and
   the fibrous sheet has a basis weight of preferably 200 g/m² or less, and more preferably 130 g/m² or less.
<47> The warming device as set forth in any one of clauses <1> to <46>,
   wherein the warming device has a lateral direction that corresponds to a longitudinal direction and a length direction that is perpendicular to the lateral direction,
   the warming device has a flexural rigidity in the lateral direction of, preferably 0.80 N or more, more preferably 1.00 N or more, and even more preferably 1.01 N or more, and
   the warming device has the flexural rigidity in the lateral direction of, preferably 1.20 N or less, more preferably 1.10 N or less, even more preferably 1.06 N or less, and yet even more preferably 1.03 N or less.
<48> The warming device as set forth in any one of clauses <1> to <47>,
   wherein the ratio of the non-crimped fibers relative to the total of the crimped fibers and the non-crimped fibers is 65 mass% or more and 75 mass% or less, and
   the fibrous sheet is configured to increase the thickness thereof by 0.35 mm or more during a period of 5 minutes after the start of heat generation by the warming device.

### Examples

Hereinafter, the present invention will be described in further detail by way of examples. However, the scope of the present invention is not limited to the examples given below.

### Example 1-1

A mixture of latently crimpable fibers that were uncrimped side-by-side fibers composed of 50 mass% ethylene-propylene copolymer and 50 mass% polypropylene (the melting point of ethylene-propylene copolymer: 160°C, the melting point of polypropylene: 165°C, crimp ratio: less than 5%, and the number of crimps: 0 crimps/cm) and non-crimped fibers composed of 100 mass% polypropylene (melting point: 165°C, crimp ratio: less than 5%, and the number of crimps: 0 crimps/cm) was supplied to a carding machine to form a mixed fiber web. Then, the obtained web was subjected to a needle punching process to obtain a needle punched non-woven fabric. Hot air at a temperature of 100°C was blown to the non-woven fabric using a through-type method to heat the non-woven fabric and crimp the latently crimpable fibers. The obtained non-woven fabric was used as a topsheet 5. The topsheet 5 had a single structure composed only of a single layer. The crimp ratio of the crimped fibers was 27.5%, and the number of crimps was 94 crimps/cm. The crimp ratio and the number of crimps of the non-crimped fibers remained unchanged even after heating. Also, as a backsheet 6, an air-through non-woven fabric made of fibers available from Kinsei Seishi Co., Ltd. was used as a single layer.

Separately from this, a heat generating element 3 for generating steam along with heat generation was produced. First, iron powder as an oxidizable metal was added to an aqueous solution prepared by dissolving salt as an electrolyte and a thickener in water, and the aqueous solution was stirred. Furthermore, as a reaction accelerator, activated carbon was added to the aqueous solution, which was then sufficiently stirred until it was uniformly dispersed. In this way, a heat generation composition in the form of a paste was obtained. The heat generation composition in the form of a paste was applied to a sheet of thin paper on which polyethylene was laminated, at a basis weight of 600 g/m². Next, as a liquid-retaining material, water absorbent polymer particles (Aqualic (registered trademark) CA available from Nippon Shokubai Co., Ltd.) were dispersed in the form of a layer on the heat generation composition at a basis weight of 70 g/m². A sheet of crepe paper (basis weight: 65 g/m²) was stacked on the absorbent polymer layer, and a heat generation portion 3a was thereby obtained. The heat generation portion 3a was cut into a size of 50 mm × 50 mm. Next, the heat generating element was interposed between a moisture permeable sheet and a non-moisture permeable sheet each cut into a size of 63 mm × 63 mm, and four sides of the sheets were heat sealed. In this way, a heat generating element 3 including the heat generation portion 3a housed in a pouch-like member 3b was obtained.

The heat generating element 3 formed to generate steam along with heat generation was interposed between the sheets 5 and 6 described above, and they were bonded. In this way, a warming device with a structure as shown in Figs. 1 to 3 was obtained.

### Examples 1-2 to 1-9

Warming devices were obtained in the same manner as in Example 1-1, except that the composition ratio of the crimped fibers and the non-crimped fibers in the topsheet 5 was changed as shown in Table 1 given below, and the fiber diameter of the crimped fibers was changed as shown in Table 1 given below.

### Comparative Example 1-1

A warming device was obtained in the same manner as in Example 1-2, except that the composition ratio of the crimped fibers and the non-crimped fibers in the topsheet 5 was changed as shown in Table 1 given below.

### Comparative Example 1-2

A warming device was obtained in the same manner as in Example 1-2, except that a topsheet 5 made of a needle punched non-woven fabric composed only of the crimped fibers was used.

### Measurement of Sheet thickness and Amount of Increase in Thickness

For each of the warming devices 1 obtained in Examples and Comparative Examples, topsheet thickness T1 (mm) measured at the start of heat generation, sheet thickness T2 (mm) measured 5 minutes after the start of heat generation, sheet thickness T3 (mm) measured 1 hour after the start of heat generation, and sheet thickness T4 (mm) measured at the end of the heat generation were obtained and calculated. The measurement was performed in an indoor environment at a temperature of 26°C and a relative humidity of 50%.

More specifically, each of the warming devices 1 obtained in Examples and Comparative Examples was housed in a package and hermetically sealed. After that, the package was opened, and only the topsheet 5 was carefully taken out from the warming device 1 and cut into a size of 1.5 cm square so as not to press the sheet in the thickness direction while exposing the warming device 1 to an oxygen-containing atmosphere such as air. The obtained piece of sheet was used as a measurement sample. The measurement sample was placed on a flat surface of a constant pressure thickness gauge (J-Type PG-11 available from TECLOCK CORPORATION). This operation was performed within two seconds after opening the package. Then, after two seconds after the opening of the package, sheet thickness was measured at three or more locations using the thickness gauge, and the arithmetic mean value of the measured thickness values was defined as sheet thickness T1 (mm) measured at the start of heat generation.

Also, sheet thickness T2 (mm) measured 5 minutes after the start of heat generation, sheet thickness T3 (mm) measured 1 hour after the start of heat generation, and sheet thickness T4 (mm) measured at the end of the heat generation were also obtained in the same manner: the topsheet 5 was carefully taken out from the warming device 1 and cut into the above-described size at each time point so as not to press the sheet, and the obtained piece of sheet was used as a measurement sample. The obtained sample was placed on the constant pressure thickness gauge, and sheet thickness was measured at three or more locations using the thickness gauge, and the arithmetic mean value of the measured thickness values obtained at each time point was defined as sheet thickness T2, T3, and T4, respectively. Also, the amounts of increase in thickness Ta (i.e., T4 - T1), Tb (i.e., T3 - T1), and Tc (i.e., T2 - T1) at each time point were also calculated. The results are shown in Table 1.

### Sensitive Evaluation

For each of the warming devices 1 obtained in Examples and Comparative Examples, a texture when the warming device 1 was touched with a hand and a fit when the warming device 1 was worn for 20 minutes covering the eyes were evaluated by expert panelists based on the following evaluation criteria. In each evaluation, the arithmetic mean value of evaluation points from the expert panelists is shown in Table 1 as a result of evaluation. When a rating of 3 points or more is given to a warming device as a result of evaluation, it can be said that the warming device has high levels of flexibility and fit.

### Texture Evaluation

5 points: The topsheet of the warming device 1 was very soft and had a very good texture.
4 points: The topsheet of the warming device 1 was soft and had a good texture.
3 points: The topsheet of the warming device 1 was flexible and the texture was satisfactory.
2 points: The topsheet of the warming device 1 was hard, and the texture was poor.
1 point: The topsheet of the warming device 1 was very hard, and the texture was very poor.

### Fit Evaluation

5 points: The warming device was sufficiently in close contact with the eyes while the warming device was worn, and the fit was very good.
4 points: The warming device was in close contact with the eyes while the warming device was worn, and the fit was good.
3 points: The warming device was held on the eyes and the vicinity of the eyes while the warming device was worn, and the fit was satisfactory.
2 points: The warming device was in less close contact with the eyes while the warming device was worn, and the fit was poor.
1 point: The warming device was not in close contact with the eyes while the warming device was worn, and the fit was very poor.

### Evaluation of Amount of Steam Generation

For each of the warming devices obtained in Examples and Comparative Examples, the total amount of stream generated for 10 minutes was measured using the above-described method. When the total amount of stream generated for 10 minutes takes a higher value, it indicates that the warming device has excellent heat generation characteristics and can generate a large amount of steam, and therefore it is possible to cause the target to be warmed to perceive both comfortable warmth and moisture. The results are shown in Table 1 given below.

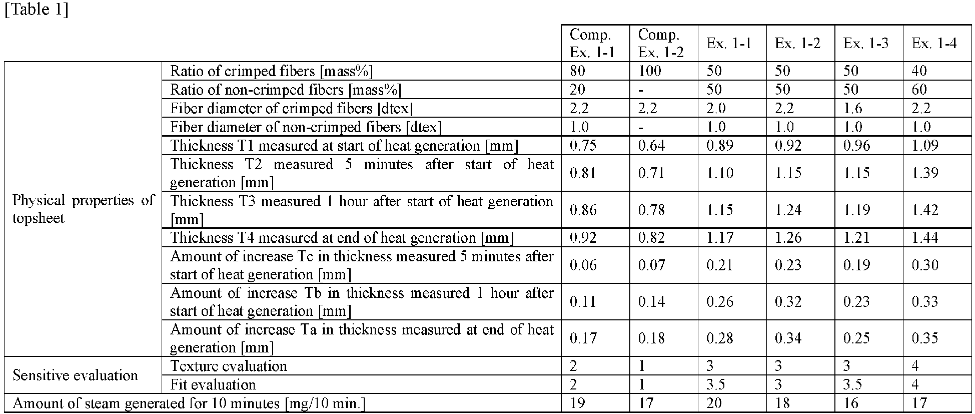

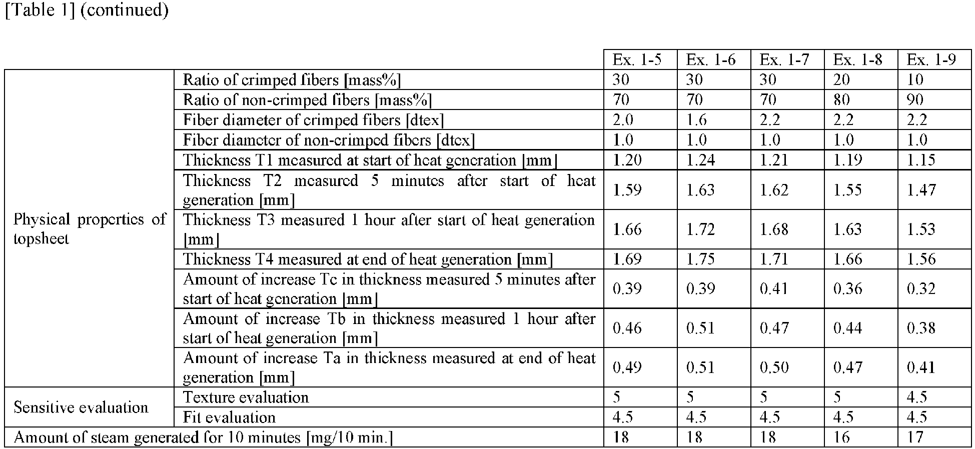

As shown in Table 1, it can be seen that the warming devices of Examples with a topsheet in which the mixing ratio between the crimped fibers and the non-crimped fibers was a predetermined ratio were more flexible and exhibited a better texture and fit when in use, as compared with the warming devices of Comparative Examples. This effect is more pronounced particularly by using a heat generating element configured in such a manner that the ratio of the crimped fibers is reduced, the ratio of the non-crimped fibers is increased, and the amount of steam generated is 10 mg/10 min or more. Accordingly, it can be seen that the warming device of the present invention has both excellent levels of flexibility and fit to the body of the wearer.

### Examples 2-1 to 2-5

Warming devices were obtained in the same manner as in Examples 1-1 to 1-9, except for the following differences. A mixture was obtained by mixing the latently crimpable fibers and the non-crimped fibers that were used in Example 1-1 in such a manner that the polypropylene content and the ethylene-propylene copolymer content satisfied the mass ratio shown in Table 2 given below. The obtained mixture was supplied to a carding machine to form a mixed fiber web. Then, the obtained web was subjected to a needle punching process to obtain a needle punched non-woven fabric. Hot air at a temperature of 100°C was blown to the non-woven fabric using a through-type method to heat the non-woven fabric and crimp the latently crimpable fibers. The obtained non-woven fabric was used as a topsheet 5. The topsheet 5 had a single-layer structure.

### Comparative Example 2-1

A warming device was obtained in the same manner as in Example 2-1, except that a needle punched non-woven fabric made only of non-crimped fibers composed of 100 mass% polypropylene was used as a topsheet 5.

### Comparative Example 2-2

A warming device was obtained in the same manner as in Example 2-1, except that only crimped fibers in which the mass ratio of polypropylene and ethylene-propylene copolymer was as shown in Table 2 given below were used in a topsheet 5.

### Measurement of Flexural Rigidity in Lateral Direction X (Longitudinal Direction)

Each of the warming devices obtained in Examples and Comparative Examples was subjected to flexural rigidity measurement. The measurement was performed in accordance with JIS K7171 (a method for testing flexural properties of plastics) using a Tensilon testing machine (RTC-1150A available from Orientec Corporation). Indenter radius R1 was set to 5.0 ± 0.1 mm, and support radius R2 was set to 5.0 ± 0.2 mm.

Next, the warming device as a measurement target was placed on two support edges with an edge span length of 15 mm so as to extend across the support edges in such a manner that the longitudinal direction of the warming device was perpendicular to the edge extending direction. At this time, the warming device 1 was placed with the topsheet 5 side facing upward. Next, the tip portion of an indenter was provided to come into a slight contact with the warming device 1. In this state, the indenter was descended with a load cell of 5 kg (range 200 cN) and a descending speed of 50 mm/min, and a load-deflection curve was obtained. The maximum value of flexural stress obtained while the indenter was descended by 10 mm is defined as flexural rigidity value (N). When the flexural rigidity value is lower, it indicates that the warming device 1 is highly flexible and has a high level of fit. The results are shown in Table 2.

### Sensitive Evaluation

For each of the warming devices 1 obtained in Examples and Comparative Examples, a texture when the warming device 1 was touched with a hand and a fit when the warming device 1 was worn were evaluated in the same manner as the above-described method. The results are shown in Table 2.

**[Table 2]**

| | | Comp. Ex. 2-1 | Comp. Ex. 2-2 | Ex. 2-1 | Ex. 2-2 | Ex. 2-3 | Ex. 2-4 | Ex. 2-5 |
|---|---|---|---|---|---|---|---|---|
| Physical properties of topsheet | Polypropylene content [mass%] | 100 | 50 | 75 | 80 | 85 | 90 | 95 |
| | Ethylene-propylene copolymer content [mass%] | 0 | 50 | 25 | 20 | 15 | 10 | 5 |
| Physical properties of warming device | Flexural rigidity in longitudinal direction (lateral direction X) [N] | 1.10 | 1.27 | 1.12 | 1.06 | 1.03 | 1.01 | 1.01 |
| Sensitive evaluation | Texture evaluation | 2 | 1 | 3 | 4 | 5 | 5 | 4.5 |
| | Fit evaluation | 2.5 | 1 | 3 | 4 | 4.5 | 4.5 | 4.5 |

As shown in Table 2, it can be seen that the warming devices of Examples with a topsheet in which the polypropylene content and the ethylene-propylene copolymer content satisfied a predetermined ratio exhibited a better texture and fit when in use, as compared with the warming devices of Comparative Examples. This is also supported by the fact that the flexural rigidity value was low. This effect is more pronounced particularly by increasing the ratio of polypropylene content. Accordingly, it can be seen that the warming device of the present invention has both excellent levels of flexibility and fit to the body of the wearer.

### Industrial Applicability

According to the present invention, a warming device that has both excellent levels of flexibility and fit to the body of the wearer is provided.

## Claims

1. A warming device comprising:
a heat generating element; and
an enclosing material that houses the heat generating element,
wherein a portion of the enclosing material that comes into contact with a target to be warmed when the warming device is in use comprises an air permeable fibrous sheet that contains crimped fibers and non-crimped fibers,
a ratio of the non-crimped fibers relative to a total of the crimped fibers and the non-crimped fibers is 50 mass% or more,
the heat generating element contains an oxidizable metal powder, a carbon material powder and water, and
the heat generating element has a function of generating steam along with heat generation.

2. The warming device according to claim 1,
wherein the non-crimped fibers have a fineness smaller than that of the crimped fibers.

3. The warming device according to claim 1 or 2,
wherein the crimped fibers have a fineness of 1.0 dtex or more and 3.0 dtex or less.

4. The warming device according to any one of claims 1 to 3,
wherein the fibrous sheet maintains a sheet configuration in which the crimped fibers and the non-crimped fibers are entangled with each other, and
the fibrous sheet includes contacting parts where the crimped fibers and the non-crimped fibers are in contact with each other.

5. The warming device according to any one of claims 1 to 4,
wherein the ratio of the non-crimped fibers relative to the total of the crimped fibers and the non-crimped fibers is 65 mass% or more and 75 mass% or less, and
the fibrous sheet is configured to increase the thickness thereof by 0.35 mm or more during a period of 5 minutes after a start of heat generation by the warming device.

6. A warming device comprising:
a heat generating element; and
an enclosing material that houses the heat generating element,
wherein a portion of the enclosing material that comes into contact with a target to be warmed when the warming device is in use comprises a fibrous sheet,
the fibrous sheet is configured to increase the thickness thereof by 0.20 mm or more and 0.70 mm or less during a period from a start to an end of heat generation by the warming device,
the heat generating element contains an oxidizable metal powder, a carbon material powder and water, and
the heat generating element has a function of generating steam along with heat generation.

7. A warming device comprising:
a heat generating element; and
an enclosing material that houses the heat generating element,
wherein a portion of the enclosing material that comes into contact with a target to be warmed when the warming device is in use comprises a fibrous sheet,
the fibrous sheet is configured to increase the thickness thereof by 0.10 mm or more and 0.60 mm or less during a period of 5 minutes after a start of heat generation by the warming device,
the heat generating element contains an oxidizable metal powder, a carbon material powder and water, and
the heat generating element has a function of generating steam along with heat generation.

8. A warming device comprising:
a heat generating element; and
an enclosing material that houses the heat generating element,
wherein a portion of the enclosing material that comes into contact with a target to be warmed when the warming device is in use comprises a fibrous sheet,
the fibrous sheet is configured to increase the thickness thereof by 0.15 mm or more and 0.70 mm or less during a period of 1 hour after a start of heat generation by the warming device,
the heat generating element contains an oxidizable metal powder, a carbon material powder and water, and
the heat generating element has a function of generating steam along with heat generation.
